# EUROPÄISCHE PATENTSCHRIFT

(11) **EP 0 647 129 B1**
(45) Veröffentlichungstag und Bekanntmachung des Hinweises auf die Patenterteilung: **06.12.1995**
(21) Anmeldenummer: 93912637.1
(22) Anmeldetag: 24.06.1993
(51) Int. Cl.: A61K 7/00, A61K 9/127

(54) **ZUBEREITUNG ZUR TOPISCHEN ANWENDUNG**
TOPICAL APPLICATION COMPOSITION
COMPOSITION POUR APPLICATION TOPIQUE

(30) Priorität: 26.06.1992 DE 4221269
(43) Veröffentlichungstag der Anmeldung: 12.04.1995
(73) Patentinhaber: LANCASTER GROUP AG, 67059 Ludwigshafen (DE)
(72) Erfinder: GROSS, Udo, D-13059 Berlin (DE); ZASTROW, Leonhard, D-65205 Wiesbaden-Nordenstadt (DE); RÖDING, Joachim, D-65207 Wiesbaden-Rambach (DE); STANZL, Klaus, White Plains, N.Y. 10605 (US)
(74) Vertreter: Walter, Wolf-Jürgen
(86) Internationale Anmeldenummer: DE9300573
(87) Internationale Veröffentlichungsnummer: WO9400097

(56) Entgegenhaltungen:
- EP-A- 0 386 680
- WO-A-89/08459
- WO-A-91/00110
- DE-A- 4 127 442

## Beschreibung

Die Erfindung betrifft eine kosmetische bzw. dermatologische Zusammensetzung mit Lichtschutzeigenschaften in einer speziellen Applikationsform, wobei fluorcarbonhaltige asymmetrische lamellare Phospholipid-Aggregate als Träger von Melanin fungieren.

Es ist bekannt, daß kurzwelliges UV-Licht (UV/A, UV/B, UV/C, Wellenlängenbereiche von 400 bis 200 nm) schädlich auf die Haut wirken kann und ein wesentlicher Faktor für die vorzeitige Hautalterung ist. In extremer Form kann die Wirkung in einer zellgenetischen Änderung von einzelnen Hautzellen bestehen und zur Bildung von Hautkarzinomen (Melanone) führen.. Diese Gefahren haben sich durch umweltbedingte Faktoren (Ozonloch) mit einer verstärkten UV-Belastung ständig erhöht. Um diesem Umstand zu begegnen ist es üblich, mittels spezieller Kosmetika und Dermatika das exponierte Hautgewebe durch Anwendung besonderer UV-Lichtschutzfilter zu schützen. Der Wirkstoff dieser Mittel beruht auf folgenden Prinzipien:
1. Absorption von UV-Licht durch Verwendung UV-aktiver organischer Verbindungen
2. Streuung von UV-Licht durch feindisperses Titandioxid oder anderen Mikropigmenten
Die Wirksamkeit dieser Systeme, die sich einfach durch Aufnahme ihrer UV-Absorptionsmaxima im Wellenlängenbereich 250-400 nm bzw. durch Streuungskurven nachweisen läßt, ist evident. Problematisch dagegen ist die biologische Unbedenklich-. keit dieser Stoffe insbesondere der möglichen chemischen Stoffänderungen unter dem Einfluß energiereicher Strahlung.

Ein weiterer kritischer Punkt ist die Eindringtiefe des UV-Filters in die Haut, wenn es als Bestandteil eines Kosmetikums oder Dermatikums verwendet wird. Der geeignete Wirkort ist der bereich zwischen Stratum corneum und Stratum basale. Um diesen Ansprüchen zu genügen, ist eine jahrelange Testung notwendig, wie sie für pharmakologische Wirkstoffe vorgeschrieben ist. Die UV-Filter werden ihrem Anwendungszweck und ihrer individuellen Wirksamkeit entsprechend in einem breiten Konzentrationsbereich zwischen 1 und 10 % in einem geeigneten Medium topisch appliziert.

So z.B. beschreibt die DE-A-3242385 (Zabotto) ein kosmetisches Mittel, das neben anderen Wirkstoffen 1,5 % Parsol Ultra (Fa. Givaudan) zur Reduzierung der Hautalterung durch Lichteinwirkung enthält.

Als natürlicher Lichtschutzwirkstoff bei höheren Lebewesen ist Melanin bekannt, das auch in menschlichen und tierischen Zellen, den Melanozyten, vorkommt. Melanin ist ein braun bis schwarz gefärbtes polymeres Pigment der Wirbeltiere, das u.a. aus der Aminosäure Tyrosin gebildet wird und sowohl Haut, Haare als auch Iris pigmentiert. Das in den Melanozyten der Haut gebildete Melanin wandert in die Basalschicht der Oberhaut und gibt dort das Pigment an die Epidermiszellen ab. Da sich Melanin als polymere Substanz kaum löst, sind die bisherigen Versuche, diesen Stoff über topische Applikationen an seinen Wirkort zu bringen, als nicht durchgreifend erfolgreich anzusehen.

Die Erfindung hat sich die Aufgabe gestellt, eine topische Anwendung von Melanin an seinem Wirkort zu ermöglichen.

Erfindungsgemäß ist eine Zubereitung zur topischen Anwendung mit Lichtschutzeigenschaften gekennzeichnet durch einen Gehalt an Melanin, das gelöst oder dispergiert ist in einem oder mehreren lipophilen Fluorcarbonen, die als asymmetrische lamellare Phospholipid-Aggregate in einem wäßrigen System zusammen mit einem Phospholipid vorliegen, mit einer Teilchengröße der Aggregate im Bereich von 200 bis 3000 nm.

Das natürlich gewonnene (wie z.B. aus Sepia officinalis) oder synthetisch erzeugte (Oxydation von Tyrosin z.B. mit H₂O₂) Melanin liegt, gelöst oder suspendiert durch das Fluorcarbon, verkapselt im Kern der asymmetrischen lamellaren Phospholipid-Aggregate vor. Die strukturelle Anordnung in den lamellaren Aggregaten ist grundsätzlich unterschieden von der wäßriger Liposomen (Vesikel). Die hydrophobe Natur des Fluorcarbons verlangt eine Polaritätsumkehr des Phospholipidmoleküls in der Weise, daß die lipophilen Fettsaurereste mit dem Fluorcarbon im Kern des Aggregates durch Dispersionskräfte wechselwirken. Auf diese Anordnung bauen sich entsprechend vorgegebener Bedingungen weitere Phospholipid-Bilayerschichten zu asymmetrischen lamellaren globulären Aggregaten auf.

Die neuartige asymmetrische Struktur wurde durch ³¹P-NMR-Untersuchungen sowie spektroskopische Untersuchungen bestätigt. Die außergewöhnliche Stabilität der Aggregate resultiert aus ihrer lamellaren Struktur und aus der gleichsinnigen Oberflächenladung.

Es können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder bicyclische Amine, Bis-(perfluoralkyl)-Ethene, Perfluorpolyether oder deren Gemische. Besonders bevorzugt sind solche Fluorcarbone wie Perfluordecalin, F-Butyltetrahydrofuran, Perfluortributylamin, Perfluoroctylbromid, Bis-Fluor(Butyl)ethen oder Bis-Fluor(Hexyl)ethen oder C₆-C₉-Perfluoralkane. Dabei liegt der Anteil an Fluorcarbonen im Bereich von 20 bis 100 % w/v, vorzugsweise im Bereich von 40 bis 100 %. Ein besonders bevorzugter Bereich ist der von 70 bis 100 % w/v.

Die Abhängigkeit der Penetrationsgeschwindigkeit und der Eindringtiefe von der Teilchengröße der Aggregate konnte durch separate Untersuchungen mit markierten verkapselten Fluorcarbonen experimentell bestimmt werden. Danach wandern kleinere Teilchen schneller und tiefer in das Hautgewebe als größere Partikel. Die Auswahl von Fluorcarbonen bzw. deren Mischungen nach ihrer Lipidlöslichkeit (dargestellt durch ihre kritische Löslichkeitstemperatur CST in n-Hexan) erlaubt als ein weiteres wichtiges Kriterium die Regelung der Verweilzeit im Gewebe. Während z. B. Perfluortributylamin (F-TBA, CST 59° C) mit einem hohen CST-Wert und schlechter Lipidlöslichkeit eine größere Verweilzeit aufweist, wird im Gegensatz dazu Perfluordecalin (PFD, CST 22° C) aber auch F-Butyltetrahydrofuran, F-Hexan und andere entsprechend schneller aus dem Gewebe abgegeben. Mit Hilfe von Fluorcarbonmischungen lassen sich auf diese Weise gezielt Systeme mit gewünschten CST-Werten d. h. Lipid- und Membranlöslichkeiten bezüglich der beabsichtigten Anwendung herstellen.

Der Gehalt der Fluorcarbone in den lamellaren Aggregaten kann entsprechend dem Anwendungzweck zwischen 1 und 100 % w/v variieren. Als Fluorcarbone kommen insbesondere in Betracht:
aliphatische geradkettige und verzweigte Alkane mit 6 bis 12 Kohlenstoffatomen, wie z. B. Perfluorhexan, Perfluoroctan, Perfluornonan;
mono- oder bicyclische Cycloalkane, die gegebenenfalls F-alkylsubstituiert sind, wie z. B. Perfluormethylcyclohexan,
Perfluordecalin;
aliphatische tertiäre Amine, N-haltige Polycyclen, wie z. B. Perfluortripropylamin, Perfluortributylamin, F-Cyclohexylmethylmorpholin;
Perfluorether, wie aliphatische Ether, F-Alkyl-Furane, bicyclische und substituierte bicyclische Ether mit 2 oder 3 Sauerstoffatomen im Molekül, wie z. B. Perfluordihexylether, Perfluorbutyltetrahydrofuran, Perfluorpolyether;
Perfluoralkylhalogenide, wie z. B. Perfluoroctylbromid, Perfluorhexylbromid, Perfluoroctylchlorid;
Bis-F(Alkyl)ethene, wie z. B. Bis-F(Butyl)ethen, Bis-F(Hexyl)ethen.

Unter dem hier verwendeten Begriff "Fluorcarbone" werden perfluorierte oder hochfluorierte Kohlenstoffverbindungen oder Gemische verstanden, die in der Lage sind, Gase wie O₂ und CO₂ zu transportieren. Teilfluorierte Kohlenwasserstoffverbindungen sind im Sinne dieser Erfindung solche, bei denen die meisten Wasserstoffatome durch Fluoratome ersetzt sind, wie z.B. die Bis-F(Alkyl)ethene, die nachweislich chemisch und biologisch inert und damit untoxisch sind. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Als Phospholipide kommen natürlich auftretende Phospholipide wie Soja- oder Eilecithin in Frage, sowie auch synthetisch herstellbare Lecithine (Phospholipide), die insgesamt als hautfreundlich und hautpflegend bekannt sind. Wegen der vorteilhaften Wirkung auf die Stabilität der asymmetrischen lamellaren Aggregate kommen vorzugsweise Phospholipidmischungen mit einem Anteil von 10 bis 99 %, vorzugsweise 30 bis 99 % , insbesondere 60 bis 90 % an Phosphatidylcholin neben weiteren natürlich auftretenden Begleitprodukten zur Verwendung. Der Phospholipidgehalt in der topischen Formulierung bewegt sich zwischen 0,5 und 20 %, vorzugsweise 10 bis 20 %.

Die Teilchengrößen der Aggregate und die Phospholipide sind so ausgewählt, daß ein Eindringen in tiefere Hautschichten, z.B. in die Epidermis oder den corialen Bereich nicht erfolgt und damit der erfindungsgemäße Lichtschutzfilter an seinen Wirkort gelangt, nachdem er das Stratum corneum penetriert hat. Die Teilchengrößen liegen im Bereich von 200 bis 3000 nm, vorzugsweise im Bereich von 250 bis 1000 nm.

Die Erfindung betrifft auch ein Verfahren zur Herstellung von Zubereitungen für die topische Anwendung, das dadurch gekennzeichnet ist, daß Melanin in einem oder mehreren Fluorcarbonen gelöst oder dispergiert wird und diese mit einem Phospholipid in einem wäßrigen System in asymmetrische lamellare Phospholipid-Aggregate, enthaltend die Fluorcarbone und Melanin, mit Teilchengrößen zwischen 200 und 3000 nm durch Homogenisierung überführt wird.

Die Löslichkeit des polymeren Melanin kann durch Zusatz von lipophilen Stoffen zum Fluorcarbon als Löslichkeitsvermittler erhöht werden. Als lipophile Stoffe sind geeignet native Öle, Triglyceride oder aliphatische Alkane, ausgewählt aus der Gruppe Olivenöl, Sojaöl, Sonnenblumenöl, Pentan, Heptan, Nonan, Dekan oder Gemische davon.

Die Homogenisierung kann nach üblichen Verfahren erfolgen, z.B. mit einem hochtourigen Rührer (12000 bis 15000 U/min), mit Ultraschall oder mittels Druckhomogenisierung in der Weise, daß die Teilchengröße gewährleistet ist.

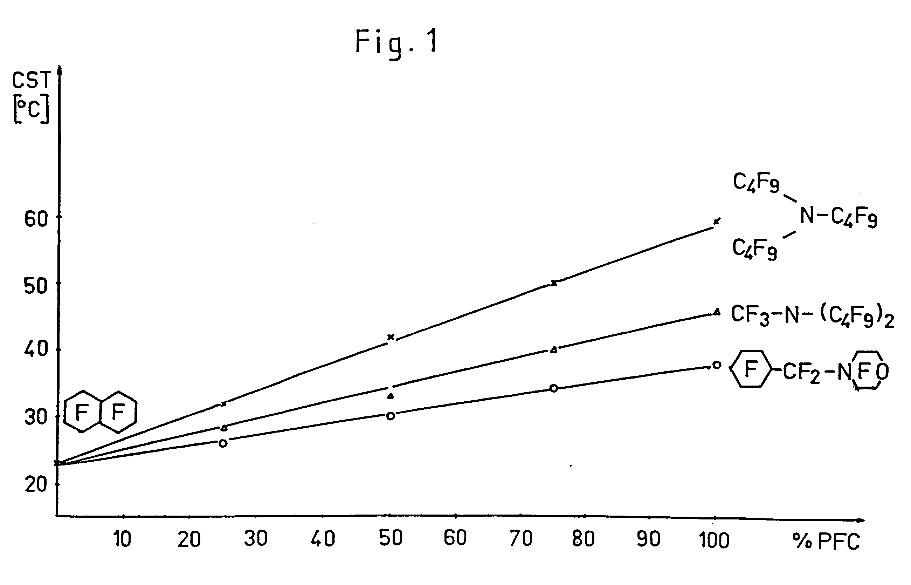

Die Erfindung soll nachstehend durch Beispiele näher erläutert werden. In der dazugehörigen Zeichnung bedeuten
Fig.1 Diagramm der kritischen Löslichkeitstemperaturen (CST) von Perfluorcarbongemischen in n-Hexan mit Perfluordecalin als Ausgangspunkt

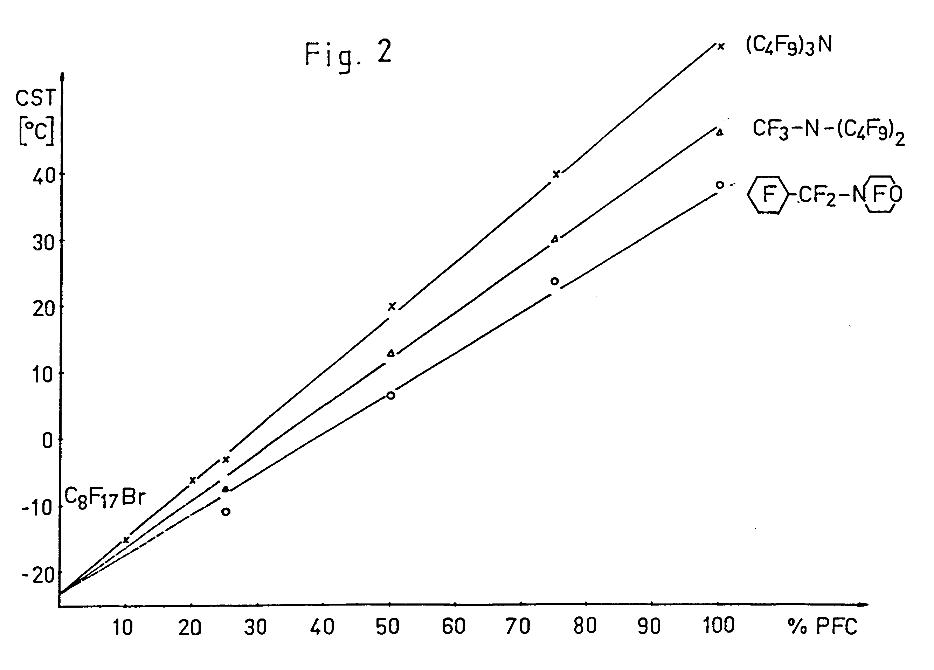

Fig.2 Diagramm der kritischen Löslichkeitstemperaturen von Perfluorcarbongemischen in n-Hexan mit F-Octylbromid als Ausgangspunkt.

In Tabelle 1 sind einige ausgewählte Fluorcarbone und ihre O₂-Löslichkeit, ihr Dampfdruck und die kritische Löslichkeitstemperatur dargestellt. Ausgehend von diesen Werten können für Gemische von Fluorcarbonen die gewünschten Charakteristika bei dem Eindringen in die Haut mit Hilfe der erfindungsgemäßen Zusammensetzung ausgewählt werden.

**Tabelle 1**

| Fluorcarbon | O₂-Löslichkeit [ml O₂/100 ml FC] | Dampfdruck P_{37°C} [mm Hg] | CST [°C] |
|---|---|---|---|
| Perfluoroctylbromid | 50 | 14 | -24,5 |
| Perfluordecalin | 40 | 12,5 | 22 |
| Bis-F(Butyl)ethen | 50 | 12,6 | 22,5 |
| F-cyclohexylmethylmorpholin | 42 | 4 | 38,5 |
| F-Tripropylamin | 45 | 18,5 | 43 |
| F-Dihexylether | 45 | 2 | 59 |
| F-Tributylamin | 40 | 1 | 59 |
| Perfluordecalin-F-Tributyl-amin 1/1 | 40 | 7 | 42 |
| Perfluorbutyltetrahydrofuran | 52 | 51 | 29 |
| F-methylcyclohexan | 57 | 180 | 8,2 |
| F-Hexan | 58 | 414 | 20 |

### Beispiel 1

Eine 10 %ige wäßrige Phospholipidlösung aus Sojalecithin und mit 40 % Phosphatidylcholin wurde zusammen mit einem Fluorcarbongemisch aus Perfluordecalin (90 %) und F-Dibutylmethylamin (10 %) und Melanin in einem Ultraschalldesintegrator unter Kühlung vermischt. Die dabei erhaltenen asymmetrischen lamellaren Phospholipidaggregate wiesen eine mittlere Teilchengröße von etwa 240 nm auf und enthielten das Melanin.
Die auf diese Weise hergestellten Aggregate wurden nach üblichen Verfahren in die nachfolgenden Verarbeitungsformen von Lichtschutzmitteln eingearbeitet.

### Beispiel 2 Emulsion (Körperlotion)

| | |
|---|---|
| Polyacrylsäure | 0,30 % |
| TEA | 0,30 % |
| p-Methylhydroxybenzoat | 0,20 % |
| p-Propylhydroxybenzoat | 0,10 % |
| Imidazolidinylharnstoff | 0,20 % |
| Na-EDTA | 0,06 % |
| Cetyl/Stearylalkohol | 1,00 % |
| Stearinsäure | 1,00 % |
| Isopropylmyristinat/-palmitat | 3,00 % |
| Paraffinum subl. | 4,00 % |
| Jojoba-Öl | 2,00 % |
| Melanin-Phospholipid-Aggregate | 10,00 % |
| Parfümöl | 1,00 % |
| demineralisiertes Wasser | q.s. |

### Beispiel 3 Emulsion (Creme)

| | |
|---|---|
| Polyacrylsäure | 0,30 % |
| Propylenglycol | 5,00 % |
| TEA | 0,30 % |
| Emulgator 1 | 6,00 % |
| Emulgator 2 | 4,50 % |
| Aloe vera | 2,00 % |
| Reisschalenöl | 1,50 % |
| Cetyl/Stearylalkohol | 1,00 % |
| Jojoba-Öl | 1,50 % |
| p-Methylhydroxybenzoat | 0,20 % |
| p-Propylhydroxybenzoat | 0,10 % |
| Imidazolidinylharnstoff | 0,20 % |
| Melanin-Phospholipid-Aggregate | 20,00 % |
| Parfümöl | 1,00 % |
| demineralisiertes Wasser | q.s. |

### Beispiel 4 Lotion

| | |
|---|---|
| Emulgatorensystem | 34,00 % |
| bestehend aus Wasser, Stabilisatoren, Polyglycerinester,Polyoxyethylenester, Isopropylpalmitat | |
| Glycerin | 5,00 % |
| MgSO₄.7H₂O | 0,50 % |
| Melanin-Phospholipid-Aggregate | 6,00 % |
| p-Methylhydroxybenzoat | 0,20 % |
| p-Propylhydroxybenzoat | 0,10 % |
| Imidazolidinylharnstoff | 0,30 % |
| Parfümöl | 1,00 % |
| demineralisiertes Wasser | qs |

### Beispiel 5 Lidpuder, gepreßt mit Lichtschutzfaktor

| | |
|---|---|
| Talkum | 40,00 % |
| Mg-Carbonat | 1,50 % |
| Mg-Stearat | 2,50 % |
| Kaolin | 2,20 % |
| Farben | 15,80 % |
| Perglanzpigmente | 21,50 % |
| Parfümöl | 1,50 % |
| Seidenprotein | 5,00 % |
| Emulsion als Prozeßvermittler | |
| Emulgator | 4,50 % |
| Siliconöl, flüchtig | 2,50 % |
| Melanin-Phospholipid-Aggregate | 4,50 % |
| Konservierung | 0,30 % |
| demineralisiertes Wasser | qs. |

## Patentansprüche

1. Zubereitung zur topischen Anwendung, gekennzeichnet durch einen Gehalt an Melanin, gelöst oder dispergiert in einem oder mehreren Fluorcarbonen, die als asymmetrische lamellare Phospholipid-Aggregate in einem wäßrigen System zusammen mit einem Phospholipid vorliegen, mit einer Teilchengröße der Aggregate im Bereich von 200 bis 3000 nm.

2. Zubereitung nach Anspruch 1, dadurch gekennzeichnet, daß der Anteil der Fluorcarbone im Bereich von 1 bis 100 % w/v liegt.

3. Zubereitung nach Anspruch 1 oder 2, dadurch gekennzeichnet, daß die Fluorcarbone aus der Gruppe ausgewählt sind, die aus aliphatischen geradkettigen und verzweigten Fluoralkanen, mono- oder bicyclischen gegebenenfalls fluoralkylsubstituierten Fluorcycloalkanen, perfluorierten aliphatischen oder bi-cyclischen Aminen, Bis-(perfluoralkyl)-ethenen, Perfluorpolyethern oder deren Gemischen besteht.

4. Zubereitung nach Anspruch 3, dadurch gekennzeichnet, daß die Fluorcarbone aus der Gruppe ausgewählt sind, die aus Perfluordecalin, F-Butyltetrahydrofuran, Perfluortributylamin, Perfluoroctylbromid, Bis-Fluor(butyl)ethen oder C₆-C₉-Perfluoralkanen besteht.

5. Zubereitung nach einem der Ansprüche 1 bis 4, dadurch gekennzeichnet, daß der Anteil an Fluorcarbonen im Bereich von 20 bis 100 % Gewicht/Volumen liegt, vorzugsweise im Bereich von 40 bis 100 %, insbesondere im Bereich von 70 bis 100 %.

6. Zubereitung nach einem der Ansprüche 1 bis 5, gekennzeichnet durch ein oder mehrere Fluorcarbone mit einer kritischen Löslichkeitstemperatur unter 50 °C, vorzugsweise unter 30 °C.

7. Zubereitung nach einem der Ansprüche 1 bis 6, dadurch gekennzeichnet, daß die Phospholipide ausgewählt sind aus der Gruppe, bestehend aus natürlichen Phospholipiden wie Sojalecithin und Eilecithin, synthetischen Phospholipiden, hydrierten Lecithinen und/oder teilhydrierten Phospholipiden.

8. Zubereitung nach einem der Ansprüche 1 bis 7, dadurch gekennzeichnet, daß Phosphatidylcholin in einem Anteil von 10 bis 99 Gew.-% vorhanden ist, vorzugsweise 30 bis 99 %, insbesondere 70 bis 90 %.

9. Zubereitung nach einem der Ansprüche 1 bis 8, dadurch gekennzeichnet, daß neben Phosphatdylcholin Lysolecithine im Konzentrationsbereich von 1 bis 10 Gew.-% vorhanden sind.

10. Zubereitung nach Anspruch 1, dadurch gekennzeichnet, daß neben Melanin und Fluorcarbon ein Löslichkeitsvermittler vorliegt, ausgewählt aus der Gruppe der nativen Öle wie Olivenöl, Sojaöl, Sonnenblumenöl, der Triglyceride oder der aliphatischen Alkane wie Pentan, Heptan, Nonan, Dekan oder Gemische innerhalb oder zwischen den Gruppen.

11. Verfahren zur Herstellung von Zubereitungen für die topische Anwendung, dadurch gekennzeichnet, daß Melanin in einem oder mehreren Fluorcarbonen gelöst oder dispergiert wird und diese mit einem Phospholipid in einem wäßrigen System in asymmetrische lamellare Phospholipid-Aggregate, enthaltend die Fluorcarbone und Melanin, mit Teilchengrößen zwischen 200 und 3000 nm durch Homogenisierung überführt wird.

12. Verfahren nach Anspruch 11, dadurch gekennzeichnet, daß Melanin und den Fluorcarbonen ein Löslichkeitsvermittler hinzugesetzt wird, ausgewählt aus der Gruppe der nativen Öle wie Olivenöl, Sojaöl, Sonnenblumenöl, der Triglyceride oder der aliphatischen Alkane wie Pentan, Heptan, Nonan, Dekan oder der Gemische innerhalb oder zwischen den Gruppen.

13. Verwendung von asymmetrischen lamellaren Phospholipid-Aggregaten zur Herstellung von Mitteln zum Einbringen von Melanin in den Hautbereich oberhalb der Epidermis, wobei die asymmetrischen lamellaren Phospholipid-Aggregate aus einem Phospholipid und einem oder mehreren Fluorcarbonen mit darin gelöstem oder dispergiertem Melanin bestehen, wobei die Teilchengröße der Aggregate im Bereich von 200 bis 3000 nm liegt.

14. Verwendung nach Anspruch 13, gekennzeichnet durch einen Phosphatidylcholingehalt der Phospholipide von 10 bis 99 Gew.-%, vorzugsweise 30 bis 99 %, insbesondere 70 bis 90 %.

## Claims

1. Preparation for topical use, characterised in that it contains melanin, dissolved or dispersed in one or more fluorocarbons, which are present as asymmetric lamellar phospholipid aggregates in an aqueous system together with a phospholipid, with a particle size of the aggregates in the range from 200 to 3000 nm.

2. Preparation according to Claim 1, characterised in that the amount of fluorocarbons is in the range from 1 to 100 % w/v.

3. Preparation according to Claim 1 or 2, characterised in that the fluorocarbons are selected from the group which consists of aliphatic straight-chain and branched fluoroalkanes, mono- or bicyclic, optionally fluoroalkyl-substituted, fluorocycloalkanes, perfluorinated aliphatic or bicyclic amines, bis-(perfluoroalkyl)ethenes, perfluoropolyethers and mixtures thereof.

4. Preparation according to Claim 3, characterised in that the fluorocarbons are selected from the group which consists of perfluorodecalin, F-butyltetrahydrofuran, perfluorotributylamine, perfluorooctyl bromide, bis-fluoro(butyl)ethene and C₆-C₉-perfluoroalkanes.

5. Preparation according to one of Claims 1 to 4, characterised in that the amount of fluorocarbons is in the range from 20 to 100 % weight/volume, preferably in the range from 40 to 100 %, in particular in the range from 70 to 100 %.

6. Preparation according to one of Claims 1 to 5, characterised by one or more fluorocarbons having a critical solubility temperature below 50°C, preferably below 30°C.

7. Preparation according to one of Claims 1 to 6, characterised in that the phospholipids are selected from the group consisting of natural phospholipids such as soya lecithin and egg lecithin, synthetic phospholipids and hydrogenated lecithins and/or partially hydrogenated phospholipids.

8. Preparation according to one of Claims 1 to 7, characterised in that phosphatidylcholine is present in an amount from 10 to 99 % by weight, preferably 30 to 99 %, in particular 70 to 90 %.

9. Preparation according to one of Claims 1 to 8, characterised in that, in addition to phosphatidylcholine, lysolecithins are present in the concentration range from 1 to 10 % by weight.

10. Preparation according to Claim 1, characterised in that, in addition to melanin and fluorocarbon, a solubiliser, selected from the group consisting of the native oils such as olive oil, soya bean oil, sunflower oil, the triglycerides or the aliphatic alkanes such as pentane, heptane, nonane or decane, and mixtures within or between the groups, is present.

11. Process for the production of preparations for topical use, characterised in that melanin is dissolved or dispersed in one or more fluorocarbons and this dispersion is converted by homogenisation with a phospholipid in an aqueous system into asymmetric lamellar phospholipid aggregates containing the fluorocarbons and melanin, having particle sizes between 200 and 3000 nm.

12. Process according to Claim 11, characterised in that a solubiliser, selected from the group consisting of the native oils such as olive oil, soya bean oil, sunflower oil, the triglycerides or the aliphatic alkanes such as pentane, heptane, nonane or decane, and mixtures within or between the groups, is added to melanin and the fluorocarbons.

13. Use of asymmetric lamellar phospholipid aggregates for the production of agents for introducing melanin into the skin region above the epidermis, the asymmetric lamellar phospholipid aggregates consisting of a phospholipid and one or more fluorocarbons containing melanin dissolved or dispersed therein, the particle size of the aggregates being in the range from 200 to 3000 nm.

14. Use according to Claim 13, characterised by a phosphatidylcholine content of the phospholipids from 10 to 99 % by weight, preferably 30 to 99 %, in particular 70 to 90 %.

## Revendications

1. Préparation pour administration par voie topique, préparation caractérisée par une teneur en mélanine, dissoute ou dispersée dans un ou plusieurs fluorocarbone(s), qui sont présents sous forme d'agrégats phospholipidiques lamellaires asymétriques dans un système aqueux avec un phospholipide, les agrégats présentant une grosseur des particules comprise entre 200 et 3000 nm.

2. Préparation selon la revendication 1, caractérisée en ce que la proportion des fluorocarbones se situe entre 1 et 100 % en poids/volume.

3. Préparation selon la revendication 1 ou 2, caractérisée en ce que les fluorocarbones sont choisis dans l'ensemble consistant en des fluoroalcanes aliphatiques linéaires et ramifiés, des fluorocycloalcanes monocycliques ou bicycliques, comportant éventuellement un ou des substituants fluoroalkyles, des amines perfluorées aliphatiques ou bicycliques, des bis(perfluoroalkyl)-éthènes, des polyéthers perfluorés ou leurs mélanges.

4. Préparation selon la revendication 3, caractérisée en ce que les fluorocarbones sont choisis dans l'ensemble consistant en de la perfluorodécaline, du F-butyltétrahydrofuranne, de la perfluorotributyl amine, du bromure de perfluoro-octyle, du bis-fluoro(butyl)-éthène ou des perfluoroalcanes en C₆ à C₉.

5. Préparation selon l'une des revendications 1 à 4, caractérisé en ce que la proportion des fluoroalcanes se situe entre 20 et 100 % en poids/volume, avantageusement entre 40 et 100 %, notamment entre 70 et 100 % en poids.

6. Préparation selon l'une des revendications 1 à 5, caractérisée par la présence d'un ou plusieurs fluorocarbones ayant une température de solubilité critique inférieure à 50°C, avantageusement inférieure à 30°C.

7. Préparation selon l'une des revendications 1 à 6, caractérisée en ce que les phospholipides sont choisis dans l'ensemble consistant en des phospholipides naturels comme de la lécithine de soja et de la lécithine d'oeuf, des phospholipides synthétiques, des lécithines hydrogénée et/ou des phospholipides partiellement hydrogénées.

8. Préparation selon l'une des revendications 1 à 7, caractérisée en ce que la phosphatidyl choline est présente en une proportion de 10 à 99 % en poids, avantageusement de 30 à 99 %, notamment de 70 à 90 %.

9. Préparation selon l'une des revendications 1 à 8, caractérisée en ce qu'en plus de la phosphatidyl choline, des lysolécithines sont présentes en une concentration de 1 à 10 % en poids.

10. Préparation selon la revendication 1, caractérisée en ce qu'en plus de la mélamine et d'un fluorocarbone, est présent un adjuvant de solubilité choisi dans l'ensemble consistant en des huiles natives ou naturelles comme l'huile d'olive, l'huile de soja, l'huile de tournesol, des triglycérides ou des alcanes aliphatiques comme le pentane, l'heptane, le nonane, le décane ou des mélanges de corps choisis à l'intérieur des groupes ou ensembles ou entre les groupes ou ensembles.

11. Procédé pour produire des préparations pour administration topique, caractérisée en ce qu'on dissout ou disperse de la mélamine dans un ou plusieurs fluorocarbones et on transforme le produit ainsi obtenu, comportant un phospholipide, par homogénéisation en un système aqueux dans des agrégats phospholipidiques lamellaires asymétriques contenant les fluorocarbones et la mélamine, et ayant des grosseurs de particules comprises entre 200 et 3000 nm.

12. Procédé selon la revendication 11, caractérisé en ce que la mélamine et les fluorocarbones sont introduits dans un adjuvant de solubilité, choisi dans le groupe ou ensemble formé par des huiles natives comme l'huile d'olive, l'huile de soja, l'huile de tournesol, des triglycérides ou des alcanes aliphatiques comme le pentane, l'heptane, le nonane, le décane ou des mélanges de corps pris à l'intérieur des groupes ou entre les groupes.

13. Utilisation d'agrégats phospholipidiques lamellaires asymétriques pour fabriquer des agents ou produits pour introduire de la mélamine dans la zone de peau située au-dessus de l'épiderme, les agrégats phospholipifiques lamellaires asymétriques consistant en un phospholipide et en un ou plusieurs fluorocarbone(s) contenant de la mélamine dissoute ou en dispersion, la grosseur des particules des agrégats se situant entre 200 et 3000nm.

14. Utilisation selon la revendication 12, caractérisée par une teneur en phosphatidyl choline des phospholipides de 10 à 99 % en poids, avantageusement de 30 à 99 %, notamment de 70 à 90 %.
